# EUROPEAN PATENT SPECIFICATION

(11) **EP 1 925 675 B1**
(45) Date of publication and mention of the grant of the patent: **03.02.2010**
(21) Application number: 07022944.8
(22) Date of filing: 06.11.2001
(51) Int. Cl.: C12P 19/38, C12N 15/09

(54) **Process for production of nucleoside compound**
Verfahren zur Herstellung einer Nukleosidverbindung
Procédé de production de composé nucléoside

(30) Priority: 06.11.2000 JP 2000337715; 14.12.2000 JP 2000380576; 22.03.2001 JP 2001082857
(43) Date of publication of application: 28.05.2008
(62) Divisional of application: 01980986.2
(73) Proprietor: Mitsui Chemicals, Inc., Tokyo 105-7117 (JP)
(72) Inventor: Araki, Tadashi, Mobara-shi Chiba 297-0017 (JP); Ikeda, Ichiro, Mobara-shi Chiba 297-0017 (JP); Takahashi, Katsuyuki, Mobara-shi Chiba 297-0017 (JP); Ito, Kiyoshi, Mobara-shi Chiba 297-0017 (JP); Asano, Tamotsu, Mobara-shi Chiba 297-0017 (JP); Nikumaru, Seiya, Mobara-shi Chiba 297-0017 (JP); Nakamura, Takeshi, Mobara-shi Chiba 297-0017 (JP); Ishibashi, Hiroki, Fukuoka 836-8610 (JP); Nagahara, Kiyoteru, Fukuoka 836-8610 (JP); Fukuiri, Yasushi, Fukuoka 836-8610 (JP)
(74) Representative: Stuart, Ian Alexander

(56) References cited:
- DATABASE WPI Week 198922 Derwent Publications Ltd., London, GB; AN 1989-161741 XP002470943 & JP 01 104190 A (AJINOMOTO KK) 21 April 1989 (1989-04-21)

## Description

### Technical Field

The present invention relates to a process for producing a nucleoside compound using nucleoside phosphorylase. Various nucleosides and analogue compounds thereof are used as a raw material for antiviral medicine, anticancer medicine, antisense medicine, etc., or as a component used in such medicines.

### Background Art

Nucleoside phosphorylase is a generic term of enzymes which give rise to phosphorolysis of the N-glycoside bond of nucleoside in the presence of phosphoric acid, and, in the case of ribonucleoside, catalyzes a reaction represented by the following formula :

ribonucleoside + phosphoric acid (salt) → nucleic acid base + ribose 1-phosphoric acid

The above enzymes are largely divided into purine nucleoside phosphorylase and pyrimidine nucleoside phosphorylase, occur widely in the biological world, and are present in the tissues of mammals, birds, fishes, etc., as well as in yeasts and bacteria. The above enzyme reaction is reversible and synthesis of various nucleoside compounds utilizing the reverse reaction has been known heretofore.

There are known, for example, processes for producing thymidine (JP-A-01-104190), 2'-deoxyadenosine (JP-A-11-137290) or 2'-deoxyguanosine (JP-A-11-137290) from 2'-deoxyribose 1-phosphoric acid and a nucleic acid base (thymine, adenine or guanine). Further, a technique was reported in Agric., Biol., Chem., Vol. 50 (1), pp. 121-126, (1986), including the steps of decomposing inosine into ribose 1-phosphoric acid and hypoxanthine through a reaction in the presence of phosphoric acid using a purine nucleoside phosphorylase derived from Enterobacter aerogenes, and producing ribavirin, as an anti-viral agent, from the ribose 1-phosphoric acid, which is isolated by an ion exchange resin and 1,2,4-triazole-3-carboxamide in the presence of the same purine nucleoside phosphorylase derived from Enterobacter aerogenes.

However, since the reaction for producing a nucleoside compound from either pentose-1-phosphoric acid or a salt thereof and a nucleoside acid base by using the above reverse reaction in the presence of the enzyme is that with an equilibrium state, the reaction has had a technical drawback of low conversion.

### Disclosure of the Invention

The object to be achieved by the present invention is to provide, a simple method for allowing a reaction to proceed at a high conversion and a means contributing to a reduction in raw material cost and an improvement of product purity, in producing a nucleoside compound industrially.

Therefore, the object of the present invention is to provide a simple and widely-applied process for producing a nucleoside compound, which comprises a step of reacting pentose-1-phosphoric acid with a nucleic acid base or a nucleic acid base analogue in the presence of nucleoside phosphorylase activity, wherein the nucleoside compound is produced at a high conversion rate.

The present inventors made an intensive study in order to achieve the above object. As a result, the present inventors found that, when a metal cation capable of forming a sparingly water-soluble salt with phosphoric acid ion is allowed to be present in the reaction mixture, the phosphoric acid ion formed as a by-product of reaction is precipitated as a sparingly water-soluble salt and removed out of the reaction system and, as a result, the equilibrium of reaction moves to a direction of nucleoside compound synthesis and a conversion rate of reaction increase.

It was also found that when a sparingly water-soluble magnesium compound such as magnesium hydroxide, is used to provide the metal cation capable of forming a sparingly water-soluble salt with phosphoric acid, various advantages are obtained. For example, since there is no increase in the salt concentration of the reaction mixture, a reduction of the reaction rate is prevented, which is caused by increase of the salt concentration in the reaction mixture. Since such a base of a metal cation can be added in one portion (total amount) before the reaction, the operation of reaction is simpler than when an aqueous solution of magnesium is added. The filtration efficiency for the reaction mixture obtained is improved strikingly.

The present inventors also found that, when magnesium hydroxide is used, a crystal of sparingly water-soluble phosphate (magnesium phosphate) formed as a by-product of reaction has a size and shape relatively suited for filtration of the reaction mixture, which enables quick filtration of the reaction mixture conducted after the completion of reaction.

According to the invention there is provided a process for producing a nucleoside compound, which comprises reacting pentose-1-phosphoric acid with a nucleic acid base or a nucleic acid base analogue in an aqueous reaction medium in the presence of nucleoside phosphorylase activity and a metal cation capable of forming a sparingly water-soluble salt with phosphoric acid, without pH adjustment, to produce a nucleoside compound, which process is **characterized in that** said metal cation is magnesium, and is added as at least one salt compound selected from magnesium hydroxide, magnesium oxide, magnesium carbonate and basic magnesium carbonate. Desirably, the pH of the aqueous reaction medium during the reaction is in a range of 7.5 to 10.0.

Desirably the process includes a step of filtering the reaction mixture obtained by the reaction, to remove the insoluble substance present in the reaction mixture to obtain a filtrate containing a nucleoside compound.

### Best Mode for Carrying Out the Invention

In the present invention, the pentose-1-phosphoric acid is a compound in which phosphoric acid is bonded to the 1-position of a polyhydroxyaldehyde or polyhydroxyketone or a derivative thereof to form an ester bond. As representative examples thereof, there can be mentioned ribose 1-phosphoric acid, 2'-deoxyribose 1-phosphoric acid, 2',3'-dideoxyribose 1-phosphoric acid and arabinose 1-phosphoric acid. However, the pentose-1-phosphoric acid is not restricted thereto.

As the polyhydroxyaldehyde or polyhydroxyketone, there can be mentioned, as natural products, aldopentoses such as D-arabinose, L-arabinose, D-xylose, L-lyxose, D-ribose and the like; ketopentoses such as D-xylulose, L-xylulose, D-ribulose and the like; and deoxysugars such as D-2-deoxyribose, D-2,3-dideoxyribose and the like. However, the polyhydroxyaldehyde or polyhydroxyketone is not restricted thereto.

The pentose-1-phosphoric acid can be produced by, for example, phosphorolysis of nucleoside compound in the presence of nucleoside phosphorylase (J. Biol. Chem. Vol. 184, 437, 1950) or anomer-selective chemical synthesis.

The nucleic acid base used in the present invention is a nitrogen atom-containing heterocyclic compound which is a structural element of a compound such as DNA, RNA or the like, and is a natural or unnatural nucleic acid base selected from the group consisting of pyrimidine, purine and base analogues. The base may be substituted with halogen atom, alkyl group, haloalkyl group, alkenyl group, haloalkenyl group, alkynyl group, amino group, alkylamino group, hydroxyl group, hydroxyamino group, aminooxy group, alkoxy group, mercapto group, alkylmercapto group, aryl group, aryloxy group or cyano group.

The halogen atom as a substituent is exemplified by chlorine, fluorine, iodine and bromine. The alkyl group is exemplified by alkyl groups of 1 to 7 carbon atoms such as methyl group, ethyl group, propyl group and the like. The haloalkyl group is exemplified by haloalkyl groups of 1 to 7 carbon atoms such as fluoromethyl, difluoromethyl, trifluoromethyl, bromomethyl, bromoethyl and the like. The alkenyl group is exemplified by alkenyl groups of 2 to 7 carbon atoms such as vinyl, allyl and the like. The haloalkenyl group is exemplified by haloalkenyl groups having an alkenyl of 2 to 7 carbon atoms, such as bromovinyl, chlorovinyl and the like. The alkynyl group is exemplified by alkynyl groups of 2 to 7 carbon atoms such as ethynyl, propynyl and the like. The alkylamino group is exemplified by alkylamino groups having an alkyl group of 1 to 7 carbon atoms, such as methylamino, ethylamino and the like. The alkoxy group is exemplified by alkoxy groups of 1 to 7 carbon atoms, such as methoxy, ethoxy and the like. The alkylmercapto group is exemplified by alkylmercapto groups having an alkyl group of 1 to 7 carbon atoms, such as methylmercapto, ethylmercapto and the like. The aryl group is exemplified by phenyl group; alkylphenyl groups having an alkyl group of 1 to 5 carbon atoms, such as methylphenyl, ethylphenyl and the like; alkoxyphenyl groups having an alkoxy group of 1 to 5 carbon atoms, such as methoxyphenyl, ethoxyphenyl and the like; alkylaminophenyl groups having an alkylamino group of 1 to 5 carbon atoms, such as dimethylaminophenyl, diethylaminophenyl and the like; halogenophenyl groups such as chlorophenyl, bromophenyl and the like; and so forth.

As specific examples of the nucleic acid base having a pyrimidine skeleton, there can be mentioned cytosine, uracil, 5-fluorocytosine, 5-fluorouracil, 5-chlorocytosine, 5-chlorouracil, 5-bromocytosine, 5-bromouracil, 5-iodocytosine, 5-iodouracil, 5-methylcytosine, 5-methyluracil (thymine), 5-ethylcytosine, 5-ethyluracil, 5-fluoromethylcytosine, 5-fluorouracil, 5-trifluorocytosine, 5-trifluorouracil, 5-vinyluracil, 5-bromovinyluracil, 5-chlorovinyluracil, 5-ethynylcytosine, 5-ethynyluracil, 5-propynyluracil, pyrimidin-2-one, 4-hydroxyaminopyrimidin-2-one, 4-aminooxypyrimidin-2-one, 4-methoxypyrimidin-2-one, 4-acetoxypyrimidin-2-one, 4-fluoropyrimidin-2-one and 5-fluoropyrimidin-2-one.

As specific examples of the nucleic acid base having a purine skeleton, there can be mentioned purine, 6-aminopurine (adenine), 6-hydroxypurine, 6-fluoropurine, 6-chloropurine, 6-methylaminopurine, 6-dimethylaminopurine, 6-trifluoromethylaminopurine, 6-benzoylaminopurine, 6-acetylaminopurine, 6-hydroxyaminopurine, 6-aminooxypurine, 6-methoxypurine, 6-acetoxypurine, 6-benzoyloxypurine, 6-methylpurine, 6-ethylpurine, 6-trifluoromethylpurine, 6-phenylpurine, 6-mercaptopurine, 6-methylmercaptopurine, 6-aminopurine-1-oxide, 6-hydroxypurine-1-oxide, 2-amino-6-hydroxypyrine (guanine), 2,6-diaminopurine, 2-amino-6-chloropurine, 2-amino-6-iodopurine, 2-aminopurine, 2-amino-6-mercaptopurine, 2-amino-6-methylmercaptopurine, 2-amino-6-hydroxyaminopurine, 2-amino-6-mehtoxypurine, 2-amino-6-benzoyloxypurine, 2-amino-6-acetoxypurine, 2-amino-6-methylpurine, 2-amino-6-cyclopropylaminomethylpurine, 2-amino-6-phenylpurine, 2-amino-8-bromopurine, 6-cyanopurine, 6-amino-2-chloropurine (2-chloroadenine) and 6-amino-2-fluoropurine (2-fluoroadenine).

As specific examples of the base analogue, there can be mentioned 6-amino-3-deazapurine, 6-amino-8-azapurine, 2-amino-6-hydroxy-8-azapurine, 6-amino-7-deazapurine, 6-amino-1-deazapurine and 6-amino-2-azapurine.

In the present invention, the nucleoside compound is a compound in which a nucleic acid base having a pyrimidine skeleton, a nucleic acid base having a purine skeleton, or a base analogue is bonded to the 1-position of the above-mentioned polyhydroxyaldehyde or polyhydroxyketone or its derivative to form an N-glycoside bond. As specific examples, there can be mentioned thymidine, deoxythymidine, deoxyuridine, deoxyguanosine, deoxyadenosine, dideoxyadenosine, trifluorothymidine, ribavirin, orotidine, uracil arabinoside, adenine arabinoside, 2-methyl-adenine arabinoside, 2-chloro-hypoxanthine arabinoside, thioguanine arabinoside, 2,6-diaminopurine arabinoside, cytosine arabinoside, guanine arabinoside, thymine arabinoside, enocitabine, gemcitabine, azidothymidine, idoxuridine, dideoxyadenosine, dideoxyinosine, dideoxycytidine, didehydrodeoxythymidine, thiadideoxycytidine, sorbidine, 5-methyl-uridine, pyrazole, thioinosine, tegafur, doxyfluridine, bredinin, nebularin, allopurinol uracil, 5-fluorouracil, 2'-aminouridine, 2'-aminoadenosine, 2'-aminoguanosine and 2-chloro-2'-aminoinosine.

In the present invention, the nucleoside phosphorylase is a generic term of enzymes which decompose the N-glycoside bond of nucleoside compound in the presence of phosphoric acid. In the present invention, the reverse reaction can be utilized. The enzyme used in the reaction may be an enzyme of any kind and any origin as long as it has an activity capable of forming an intended nucleoside compound from a corresponding pentose-1-phosphoric acid and a nucleic acid base or a nucleic acid base analogue. The enzyme is largely divided into a purine type and a pyrimidine type. As known enzymes of purine type, there can be mentioned, for example, purine nucleoside phosphorylase (EC 2.4.2.1) and guanosine phosphorylase (EC 2.4.2.15); as known enzymes of pyrimidine type, there can be mentioned, for example, pyrimidine nucleoside phosphorylase (EC 2.4.2.2), uridine phosphorylase (EC 2.4.2.3), thymidine phosphorylase (EC 2.4.2.4) and deoxyuridine phosphorylase (EC 2.4.2.23).

In the present invention, there is no particular restriction as to the microorganisms having nucleoside phosphorylase activity, as long as the microorganisms express at least one nucleoside phosphorylase selected from the group consisting of purine nucleoside phosphorylase (EC 2.4.2.1), guanosine phosphorylase (EC 2.4.2.15), pyrimidine nucleoside phosphorylase (EC 2.4.2.2), uridine phosphorylase (EC 2.4.2.3), thymidine phosphorylase (EC 2.4.2.4) and deoxyuridine phosphorylase (EC 2.4.2.23).

As preferred specific examples of such a microorganism, there can be mentioned microorganism strains included in Norcardia genus, Microbacterium genus, Corynebacterium genus, Brevibacterium genus, Cellulomonas genus, Flabobacterium genus, Kluyvere genus, Mycobacterium genus, Haemophilus genus, Mycoplana genus, Protaminobacter genus, Candida genus, Saccharomyces genus, Bacillus genus, thermophilic Bacillus genus, Pseudomonas genus, Micrococcus genus, Hafnia genus, Proteus genus, Vibrio genus, Staphyrococcus genus, Propionibacterium genus, Sarcina genus, Planococcus genus, Escherichia genus, Kurthia genus, Rhodococcus genus, Acinetobacter genus, Xanthobacter genus, Streptomyces genus, Rhizobium genus, Salmonella genus, Klebsiella genus, Enterobacter genus, Erwinia genus, Aeromonas genus, Citrobacter genus, Achromobacter genus, Agrobacterium genus, Arthrobacter genus and Pseudonocardia genus.

By examining the molecular biological properties, amino acid sequences, etc. of the nucleoside phosphorylases of the above-mentioned microorganism strains based on the recent progress of molecular biology and genetic engineering, it has become possible and even relatively easy to take out a gene for the protein from a microorganism strain, construct a recombinant plasmid, into which the gene and a regulatory region necessary for expression have been inserted, introduce the plasmid into a particular host, and produce recombinant cells in which the protein has been expressed. In view of such a technical standard, even such recombinant cells obtained by introducing the gene for the nucleoside phosphorylase into a particular host are included in the microorganism used in the present invention, in which nucleoside phosphorylase is expressed.

The regulatory regions defined here include promoter sequences, which include operator sequences for control of transcription, ribosome binding sequences (SD sequences) and sequences for formation of transcription. Concrete examples of the promoter sequences include trp promoter form tryptophan operon, lac promoter form lactose operon, P_{L} promoter and P_{R} promoter from lambda phage, promoters for gluconic acid synthesizing enzyme (gnt) of B. subtilis, alkaline protease promoters (aprs), neutral protease promoters (nprs) and α-amylase promoters (amys). Promoters such as tac promoter, which are modified or designed individually, may also be used. As the ribosome binding sequences, although the sequences originated from E. coli or B. subtilis can be mentioned, this sequence is not specially limited provided that the sequence can have its function in an E. coli or B. subtilis host. A consensus sequence consisting of 4 or more nucleotides in a sequence complementary to the 3' terminal region of 16S ribosome RNA may be synthesized and used. The transcription terminating sequence is not always necessary, but those independent of ρ-factor, such as lipoprotein terminator, trp operon terminator may be used. Regarding the order of arrangement of these regulatory sequences, a promoter sequence, a ribosome binding sequence, a gene encoding the nucleoside phosphorylase and a transcription termination sequence are preferably arranged in this order.

Examples of plasmids include pBR322, pUC18, Bluescript II SK(+), pKK223-3, pSC101, each of which has a region to make its self-replication possible in E. coli, pUB110, pTZ4, pC194, ρ-11, φ-1 and φ-105, each of which has a region to make its self-replication possible in B. subtilis. As examples of plasmids which can replicate themselves in two different hosts, pHV14, YEp7 and pBS7 may be used as a vector.

As the particular host mentioned here, there can be cited Escherichia coli as a representative example, as used in Examples described later. However, the host is not restricted thereto and includes other microorganism strains such as Bacillus genus cells (e.g. Bacillus subtilis), yeasts, and actinomycetes.

In the present invention, as the nucleoside phosphorylase or the microorganism having such an enzyme activity, there can be used a commercial enzyme, microbial cells having the enzyme activity, a product obtained by treatment of cells, and an immobilized product thereof. The product obtained by treatment of cells is, for example, acetone-dried cells or a cell disruption product prepared by mechanical disruption, ultrasonic disruption, freeze-thawing, pressure-reduced pressure treatment, osmosis treatment, autolysis, cell wall decomposition, or surfactant treatment. The product may, as necessary, be purified by ammonium sulfate precipitation, acetone precipitation, or column chromatography.

When the magnesium compound is put in an aqueous solution of a nucleic acid base or a nucleic acid base analogue and is added to a reaction medium, the amount of the magnesium cation added to the aqueous solution is suitably 0.5 to 20 moles, preferably 1 to 4 moles per mole of the nucleic acid base or the nucleic acid base analogue.

Incidentally, when the components required for a reaction are mixed at one time, there may arise solidification, etc. of reaction mixture depending upon the use amounts of the components. In such a case, problems of such solidification, etc. can be prevented by adding, after the start of reaction, at least one component selected from (1) pentose-1-phosphoric acid, (2) nucleic acid base or nucleic acid base analogue and (3) metal cation, to an aqueous reaction medium which is in the middle of reaction. Here, "the start of reaction" indicates a state in which at least part of the addition amounts of pentose-1-phosphoric acid and nucleic acid base or nucleic acid base analogue has been added in the presence of nucleoside phosphorylase activity.

Each component added after the start of reaction can be added at one time as mentioned above, or in two or more portions batchwise, or continuously, or in a combination of batchwise addition and continuous addition. When the component added after the start of reaction is more than one, at least one component selected from the above-mentioned three components can be added in portions. That is, when the component added after the start of reaction is more than one, an appropriate addition method can be selected for each individual component.

Since the magnesium compounds used in the invention are, per se, sparingly soluble in water, the total amount to be added by the completion of reaction can be added to an aqueous reaction medium before the start of reaction, and control of the pH of the reaction mixture may not be necessary; therefore, the use of such compounds is advantageous, particularly magnesium hydroxide.

The aqueous reaction medium used in the present process can be any reaction medium which is composed mainly of water and with which a reaction using an enzyme activity, which is intended by the present invention, proceeds and an effect of metal cation addition is exhibited. As such an aqueous reaction medium, there can be mentioned water or an aqueous reaction medium obtained by adding a water-soluble organic solvent to water. The organic solvent can be exemplified by lower alcohols (e.g. methanol and ethanol), acetone, dimethyl sulfoxide and mixtures thereof. There is no particular restriction as to the amount of the solvent used, and the amount may be such that a reaction proceeds favorably. The amount of the organic solvent relative to water can be preferably 0.1% to 70% by weight. The reaction mixture using such an aqueous reaction medium may be used as a buffer solution in order to secure the stability of reaction. As such a buffer solution, there can be used a known buffer solution which can be used for the enzyme reaction of the present invention.

In the synthesis reaction of nucleoside compound according to the present invention, the reaction conditions, such as appropriate pH and temperature, can be selected depending upon the kinds and properties of pentose-1-phosphoric acid and nucleic acid base as substrate, nucleoside phosphorylase or microorganism having the enzyme activity, as reaction catalyst, and the magnesium compound. Ordinarily, however, the pH can be selected in a range of 5 to 11, preferably 7.5 to 10.0, more preferably 8.0 to 10 and the temperature can be selected in a range of the freezing point to 80°C, preferably 10 to 60°C. When the pH is out of the control range, it is possible to invite reduction of the conversion rate due to stability of intended product or substrate, reduction in enzyme activity, no formation of sparingly water-soluble salt with phosphoric acid, etc. When pH change arises in the middle of reaction, it is possible to add as necessary an acid such as hydrochloric acid or sulfuric acid, or an alkali such as sodium hydroxide or potassium hydroxide. In fact, pH control of the reaction mixture may be unnecessary owing to the use of a sparingly water-soluble magnesium compound, particularly magnesium hydroxide.

The concentration of pentose-1-phosphoric acid and the nucleic acid base used in the reaction is appropriately about 0.1 to 1,000 mM. The molar ratio of the two is such that the amount of the nucleic acid base is 0.1 to 10 moles per mole of pentose-1-phosphoric acid or its salt. The amount is preferably 0.95 mole or less in view of the reaction conversion.

The reaction time differs depending upon the raw materials, the kind of aqueous solvent and the temperature of reaction, but is 1 minute to 78 hours, preferably 30 minutes to 24 hours.

The magnesium compound may be added in an amount of 0.1 to 10 moles, preferably 0.5 to 5 moles per mole of the pentose-1-phosphoric acid used in the reaction. Although, in a reaction under a high concentration, the nucleic acid base as substrate and the nucleoside compound as product may be present in the reaction mixture without being fully dissolved, the present invention is applicable even in such a case.

In the present invention, in order to add at least one component selected from pentose-1-phosphoric acid or its salt, a magnesium compound capable of forming a sparingly water-soluble salt with phosphoric acid or its salt, and a nucleic acid base to a reaction medium so that no solidification of reaction mixture occurs, the following methods can be mentioned as representative methods. Incidentally, the present invention is not restricted to the following methods and, as long as no solidification of reaction mixture occurs, there is no particular restriction as to the timing and times of addition of at least one component selected from pentose-1-phosphoric acid or its salt, a magnesium compound, and a nucleic acid base.
1. A method comprising the steps of feeding pentose-1-phosphoric acid or its salt and a nucleic acid base or its analogue in one portion to start a reaction, accumulating a nucleoside compound up to an appropriate concentration, and then adding the magnesium compound in one portion. In this case, the appropriate concentration of nucleoside compound accumulated is preferably 10 mM or more, more preferably 30 mM or more.
2. A method comprising the steps of feeding pentose-1-phosphoric acid or its salt and a nucleic acid base or its analogue in one portion to start a reaction, and then adding the magnesium compound stepwise or continuously so as to match the concentration of nucleoside compound accumulated. In this case, the addition of the magnesium compound can be started when the nucleoside compound has accumulated in an amount of preferably 10 mM or more, more preferably 30 mM or more.
3. A method comprising the steps of adding pentose-1-phosphoric acid or its salt and the magnesium compound in one portion, and then adding a nucleic acid base or its analogue stepwise or continuously so that no solidification of reaction mixture occurs. In this case, the amount of the nucleic acid base or its analogue added initially is preferably 30 mM or less, more preferably 10 mM or less.
4. A method comprising the steps of adding a nucleic acid base or its analogue and the magnesium compound in one portion, and then adding pentose-1-phosphoric acid or its salt stepwise or continuously so that no solidification of reaction mixture occur. In this case, the amount of the pentose-1-phosphoric acid or its salt added initially is preferably 30 mM or less, more preferably 10 mM or less.
5. A method comprising the steps of adding pentose-1-phosphoric acid or its salt, the magnesium compound, and a nucleic acid base or its analogue, into an enzyme solution stepwise or continuously. In this case, the amount of the pentose-1-phosphoric acid or its salt, or the nucleic acid base or its analogue added at the start of reaction is preferably 30 mM or less, more preferably 10 mM or less.

An ordinary method such as concentration, crystallization, dissolution, electrodialysis, adsorption and desorption by ion exchange resin or active carbon, and the like can be used to isolate the nucleoside compound thus produced from the reaction mixture.

### [Examples]

The present invention is described below by way of Examples and Comparative Examples. However, the present invention is in no way restricted by these Examples, etc.

### Preparation of Enzyme Solution

A DNA of Escherichia coli chromosome was produced as follows. An Escherichia coli K-12/XL-10 strain (Stratagene Co.) was inoculated in 50 ml of a LB medium and cultured at 37°C overnight. The resulting cells were collected and subjected to bacteriolysis with a solution for the bacteriolysis containing 1 mg/ml of lysozyme. The resulting solution was subjected to a phenol treatment and then to an ethanol treatment by an ordinary method to precipitate a DNA. The DNA precipitate was recovered by winding it round a glass rod, washed and used for PCR.

As primers for PCR, there were used two oligonucleotides having base sequences shown by the following sequence Nos. 1 and 2, designed based on the base sequences of known deoD gene of Escherichia coli [GenBank accession No. AE000508 (code region: base Nos. 11531-12250)] (the oligonucleotides had been synthesized by Hokkaido System Science K.K. on consignment).
Sequence No. 1: gtgaattcac aaaaaggata aaacaatggc
Sequence No. 2: tcgaagcttg cgaaacacaa ttactcttt

These primers have, at around respective 5' terminal and 3' terminal, EcoRI and Hind III restriction endonuclease recognition sequences.

The above-obtained DNA of Escherichia coli chromosome was fully digested by the restriction endnuclease Hind III. 0.1 ml of a PCR mixture containing 6 ng/µl of the digested DNA and 3 µm each of the above primers was subjected to 30 cycles of a reaction (each cycle consisted of denaturation: 96°C for 1 minute, annealing: 55°C for 1 minute, and extension reaction: 74°C for 1 minute).

The reaction product and Plasmid pUC 18 (Takara Shuzo Co., Ltd.) were digested by EcoRI and Hind III and ligated using Ligation High (TOYOBO CO.., LTD.), to obtain a recombinant plasmid and, using the recombinant plasmid, Escherichia coli DH5α was transformed. The transformed strain was cultured in a LB agar medium containing 50 µg/ml of ampicillin (Am) and X-Gal (5-bromo-4-chloro-3-indolyl-β-D-galactoside), to obtain a Am-resistant transformed strain as a white colony.

A plasmid having an intended DNA segment inserted was extracted from the thus-obtained transformed strain and named as pUC-PNP 73. The thus-obtained transformed strain was named as Escherichia coli MT-10905.

The Escherichia coli MT-10905 strain was subjected to shake culture at 37°C overnight in a LB medium containing 50 µg/ml of Am. The resulting culture mixture was centrifuged at 13,000 rpm for 10 minutes to collect cells. The cells were suspended in 10 ml of a 10 mM Tris-hydrochloric acid buffer solution (pH 8.0) and disrupted by an ultrasonic wave. The resulting product was used as an enzyme source.

All the nucleoside compounds produced in the following Examples, etc. were quantitatively determined by high-performance liquid chromatography under the following analytical conditions.
Column: YMC-Pack ODS-A514 300x6.0 mm (I.D.) (YMC Co., Ltd.)
Column temperature: 40°C
Pump flow rate: 1 ml/min
Detection: UV 254 nm
Eluate: Phosphoric acid was added, for pH adjustment to 3.5, to an aqueous solution (2,700 ml) containing 20 mM of ammonium acetate; methanol (300 ml) was added; and mixing and degassing were conducted.
Internal standard substance: Nicotinic acid

### Reference Example 1

### Synthesis of di(ammonium) salt of 2-deoxyribose 1-phosphoric acid

Di(monocyclohexylammonium) salt of 2-deoxyribose 1-phosphoric acid (1 g, 2.42 mmol, a product of SIGMA) was suspended in methanol (10 g). Ammonia gas (0.62 g, 36.5 mmol) was blown thereinto and stirring was conducted at 50°C for 2 hours. The resulting reaction mass was filtered at 30°C, followed by washing twice with methanol (5 g) and drying, to obtain intended di(ammonium) salt of 2'-deoxyribose 1-phosphoric acid (0.57 g) at a yield of 95%.
¹H-NMR (D₂O, 270 MHz) d 5.56 (s,1H), 4.03 (m,2H), 3.52 (dd, J=3.3, 12.2 Hz, 1H), 3.41 (dd, J=5.3, 12.2Hz, 1H), 2.17 (m, 1H), 1.87 (d, J=13.9 Hz, 1H), MS (APCI) m/z 213 (M-H)

### Example 1

### Synthesis of 2'-deoxyadenosine

To pure water (13 g) were added di(ammonium) salt of 2-deoxyribose 1-phosphoric acid (1.67 g, 6.7 mmol), adenine (0.87 g, 6.4 mmol) and magnesium hydroxide (0.59 g, 10.1 mmol). To the resulting reaction mass was added 0.05 ml of an enzyme solution, and a reaction was conducted at 45°C for 3 hours with stirring. The reaction yield was 98%. The resulting reaction mass was heated to 70°C to dissolve the formed 2'-deoxyadenosine; then, the insoluble magnesium phosphate formed by the reaction was removed by filtration using a 5A filter paper, followed by washing with water (5 g); a clear filtrate (18.2 g) was obtained from the reaction mass at a yield of 99%. The filtration rate was 1,000 kg/m³/hr and the ratio of loss of 2'-deoxyadenosine into cake was 1%. The above-obtained filtrate was subjected to crystallization at 5°C for 2 hours. The resulting crystals were subjected to filtration, washed with water (5 g), and vacuum-dried to obtain intended 2'-deoxyadenosine (1.6 g) at a yield of 93%.

### Comparative Example 1

### Synthesis of 2'-deoxyadenosine

In pure water (13 g) were suspended di(ammonium) salt of 2-deoxyribose 1-phosphoric acid (1.67 g, 6.7 mmol), adenine (0.87 g, 6.4 mmol) and 0.05 ml of an enzyme solution. Thereto were dropwise added, at 45°C in 2 hours, an aqueous solution (3.7 g) of calcium chloride monohydrate (1.48 g) and a 4% aqueous sodium hydroxide solution (20.1.g) simultaneously. After the completion of the dropwise addition, the reaction mass was subjected to a reaction at the same temperature for 3 hours. The reaction yield was 97%. The resulting reaction mass was heated to 70°C to dissolve the formed 2'-deoxyadenosine; then, the insoluble calcium phosphate formed by the reaction was removed by filtration using a 5A filter paper, followed by washing with water (5 g); a clear filtrate (28.2 g) was obtained from the reaction mass at a yield of 85%. The filtration rate was 50 kg/m³/hr and the ratio of loss of 2'-deoxyadenosine into cake was 15%. The above-obtained filtrate was subjected to crystallization at 5°C for 2 hours. The resulting crystals were subjected to filtration, washed with water (5 g), and vacuum-dried to obtain intended 2'-deoxyadenosine (1.3 g) at a yield of 75%.

### Example 2

### Synthesis of 2'-deoxyadenosine

To pure water (13 g) were added di(ammonium) salt of 2-deoxyribose 1-phosphoric acid (1.67 g, 6.7 mmol), adenine (0.87 g, 6.4 mmol) and magnesium hydroxide (0.59 g, 10.1 mmol). To the resulting reaction mass was added the enzyme solution (0.05 ml) obtained as described above, and reaction was allowed to take place at 45°C for 3 hours with stirring. After 3 hours, the reaction mass was analyzed by HPLC, which indicated formation of intended 2'-deoxyadenosine at a reaction yield of 98%. The pH during the reaction was 8 to 10.

### Example 3

### Synthesis of 2'-deoxyguanosine

To pure water (15 g) were added di(ammonium) salt of 2-deoxyribose 1-phosphoric acid (1.67 g, 6.7 mmol), guanine (0.92 g, 6.1 mmol) and magnesium hydroxide (0.59 g, 10.1 mmol). To the resulting reaction mass was added the enzyme solution (0.2 ml) obtained as described above, and a reaction was allowed to take place at 50°C for 6 hours with stirring. After 6 hours, the reaction mass was analyzed by HPLC, which indicated formation of intended 2'-deoxyguanosine at a reaction yield of 98%. The pH during the reaction was 8 to 10.

### Example 4

### Synthesis of 2'-deoxyadenosine

To pure water (13 g) were added di(ammonium) salt of 2-deoxyribose 1-phosphoric acid (1.67 g, 6.7 mmol), adenine (0.87 g, 6.4 mmol) and magnesium hydroxide (0.59 g, 10.1 mmol). To the resulting reaction mass was added the enzyme solution (0.05 ml) obtained as described above, and a reaction was allowed to take place at 45°C for 3 hours with stirring while the pH was controlled at 8.5 using acetic acid. After 3 hours, the reaction mass was analyzed by HPLC, which indicated formation of intended 2'-deoxyadenosine at a reaction yield of 98%.

### Comparative Example 2

### Synthesis of 2'-deoxyadenosine

To pure water (13 g) were added di(ammonium) salt of 2-deoxyribose 1-phosphoric acid (1.67 g, 6.7 mmol), adenine (0.87 g, 6.4 mmol) and magnesium chloride hexahydrate (2.05 g, 10.1 mmol). To the resulting reaction mass was added the enzyme solution (0.05 ml) obtained as described above, and a reaction was allowed to take place at 45°C for 3 hours with stirring. After 3 hours, the reaction mass was analyzed by HPLC, which indicated formation of intended 2'-deoxyadenosine at a reaction yield of 65%. The pH during the reaction was 6 to 7.5.

### Comparative Example 3

### Synthesis of 2'-deoxyguanosine

To pure water (15 g) were added di(ammonium) salt of 2-deoxyribose 1-phosphoric acid (1.67 g, 6.7 mmol), guanine (0.92 g, 6.1 mmol) and magnesium chloride hexahydrate (2.05 g, 10.1 mmol). To the resulting reaction mass was added the enzyme solution (0.2 ml) obtained as described above, and a reaction was allowed to take place at 50°C for 3 hours with stirring. After 3 hours, the reaction mass was analyzed by HPLC, which indicated formation of intended 2'-deoxyguanosine at a reaction yield of 65%. The pH during the reaction was 6 to 7.5.

### Comparative Example 4

### Synthesis of 2'-deoxyadenosine

Reactions wwere conducted in the same manner as in Example 4 while the pH during the reaction was controlled using acetic acid, ammonia or NaOH. The yields are shown in the following Table 1. It is seen from the results that a reduction in yield takes place at a pH of lower than 7.5 and also at a pH of higher than 10.5.

**Table 1 Change of yield by change of pH**

| | | | | | |
|---|---|---|---|---|---|
| pH during reaction | 7.2 | 8.0 | 9.0 | 9.5 | 10.6 |
| Reaction yield | 83 | 99 | 99 | 99 | 22 |

### Comparative Example 5

### Synthesis of 2'-deoxyadenosine

Reactions were conducted in the same manner as in Example 2 or Comparative Example 2 with the pH during reaction controlled at 9.0 using ammonia, wherein the amount of pure water added was changed. The change in reaction yield by change in pure water addition amount is shown in the following Table 2. It is seen from the table that when a chloride salt is used, there is no increase in reaction yield owing to the influence of formed ammonium chloride unless a low reaction concentration is employed.

**Table 2**

| Magnesium salt | Magnesium chloride | | | Magnesium hydroxide | | |
|---|---|---|---|---|---|---|
| Addition amount of pure water | 30 g | 20 g | 13 g | 30 g | 20 g | 13 g |
| Reaction yield | 98 | 90 | 80 | 99 | 99 | 98 |

### Industrial Applicability

In the present invention, in producing a nucleoside compound from pentose-1-phosphoric acid and a nucleic acid base using nucleoside phosphorylase or a microorganism having the enzyme activity, a magnesium compound capable of forming a sparingly water-soluble salt with phosphoric acid is added; thereby, the phosphoric acid generated as a by-product is removed from the reaction system and a high reaction yield unobtainable with the prior art can be achieved. Further, it is possible to produce a nucleoside compound at a high yield efficiently without inviting the high viscosity or solidification of reaction mixture. With these advantages, the present invention provides a large cost reduction in industrial application and has a big significance.

When magnesium hydroxide is used, there are various advantages such as the following, because magnesium hydroxide has a low solubility in water.
(1) There is substantially no pH change of reaction mixture during reaction; therefore, deactivation of enzyme is prevented (use of water-soluble metal compound other than magnesium hydroxide results in a slight pH shift of reaction mixture to alkaline side during reaction).
(2) There is no increase in salt concentration of reaction mixture, associated with reaction; therefore, hindrance of reaction caused by increase in salt concentration is prevented and further a reaction in high concentration is possible.
(3) The magnesium compound can be added in one portion, before reaction. Further, use of a magnesium compound gives a strikingly improved filtration rate in the step of filtration of reaction mixture after the completion of reaction, which is very useful in industrial application.

## Claims

1. A process for producing a nucleoside compound, which comprises reacting a pentose-1-phosphoric acid with a nucleic acid base or a nucleic acid base analogue in an aqueous reaction medium in the presence of nucleoside phosphorylase activity and a metal cation capable of forming a sparingly water-soluble salt with phosphoric acid, without pH adjustment, to produce a nucleoside compound, which process is **characterized in that** said metal cation is magnesium cation and is added as at least one compound selected from magnesium hydroxide, magnesium oxide, magnesium carbonate and basic magnesium carbonate.

2. A process for producing a nucleoside compound according to Claim 1, **characterized in that** the pH of the aqueous reaction medium during the reaction is in a range of 7.5 to 10.0.

3. A process for producing a nucleoside compound according to Claim 1, **characterized by** comprising a step of filtering the reaction mixture obtained by the reaction, to remove the insoluble substance present in the reaction mixture to obtain a filtrate containing a nucleoside compound.

## Patentansprüche

1. Verfahren zur Herstellung einer Nukleosidverbindung, dass das Umsetzen einer Pentose-1-phosphorsäure mit einer Nukleinsäurebase oder einem Analogen der Nukleinsäurebase in einem wässerigen Reaktionsmedium in Gegenwart von Nukleosidphosphorylaseaktivität und einem Metallkation, das in der Lage ist, ein schwerlösliches wasserlösliches Salz mit der Phosphorsäure ohne Einstellung des pH-Wertes zu bilden, um eine Nukleosidverbindung zu bilden, wobei das Verfahren charakterisiert ist, dadurch dass das Metallkation ein Magnesiumkation ist und das als wenigstens eine Verbindung zugefügt wird, die ausgewählt wird aus Magnesiumhydroxid, Magnesiumoxid, Magnesiumcarbonat und basischen Magenesiumcarkonat, umfasst.

2. Verfahren zur Herstellung einer Nukleosidverbindung nach Anspruch 1, **dadurch gekennzeichnet, dass** der pH des wässerigen Reaktionsmediums während der Reaktion im Bereich von 7,5 bis 10,0 liegt.

3. Verfahren zur Herstellung einer Nukleosidverbindung nach Anspruch 1, das **gekennzeichnet ist dadurch**, dass es einen Schritt zur Filtration der Reaktionsmischung umfasst, die **durch** die Reaktion erhalten wird, um die unlösliche Substanz zu entfernen, die in der Reaktionsmischung vorhanden ist, um ein Filtrat zu erhalten, dass eine Nukleosidverbindung enthält.

## Revendications

1. Procédé de production d'un composé nucléoside, qui comprend la réaction d'un acide pentose-1-phosphorique avec une base d'acide nucléique ou un analogue de base d'acide nucléique dans un milieu de réaction aqueux en présence d'une activité de nucléoside phosphorylase et d'un cation métallique apte à former un sel difficilement soluble dans l'eau avec l'acide phosphorique, sans ajustement du pH, pour produire un composé nucléoside, ledit procédé étant **caractérisé en ce que** ledit cation métallique est un cation de magnésium et est ajouté comme au moins un composé sélectionné parmi magnésium hydroxyde, magnésium oxyde, magnésium carbonate et magnésium carbonate basique.

2. Procédé de production d'un composé nucléoside selon la revendication 1, **caractérisé en ce que** le pH du milieu de réaction aqueux durant la réaction est dans la plage de 7,5 à 10,0.

3. Procédé de production d'un composé nucléoside selon la revendication 1, **caractérisé en** comprenant une étape de filtration du mélange de réaction obtenu par la réaction, pour supprimer la substance insoluble présente dans le mélange de réaction afin d'obtenir un filtrat contenant un composé nucléoside.


### SEQUENCE LISTING

<110> Mitsui Chemicals, Inc.
<120> Process for Production of Nucleoside Compound
<130> IS/FP6144679
<140> EP 01980986.2
   <141> 2001-11-06
<150> PCT/JP01/09701
   <151> 2001-11-06
<150> JP20000337715
   <151> 2000-11-06
<150> JP20010082857
   <151> 2001-03-22
<150> JP20000380576
   <151> 2000-12-14
<160> 2
<170> PatentIn version 3.1
<210> 1
   <211> 30
   <212> DNA
   <213> Artificial sequence
<220>
   <221> source
   <223> /note = "Description of artificial sequence: Oligonucleotide to act as a PCR primer for cloning of purine nucleoside phosphorylase of E. coli K12"
<400> 1
   gtgaattcac aaaaaggata aaacaatggc 30
<210> 2
   <211> 29
   <212> DNA
   <213> Artificial sequence
<220>
   <221> source
   <223> /note = "Description of artificial sequence: Oligonucleotide to act as a PCR primer for cloning of purine nucleoside phosphorylase of E. coli K12"
<400> 2
   tcgaagcttg cgaaacacaa ttactcttt 29